(19) Europäisches Patentamt
European Patent Office
Office européen des brevets

(11) Publication number: **0 359 430 B1**

(12) **EUROPEAN PATENT SPECIFICATION**

(45) Date of publication of patent specification: **10.05.95 Bulletin 95/19**

(51) Int. Cl.$^6$: **G03F 7/029,** C07D 251/22, C07D 251/16

(21) Application number: **89308688.4**

(22) Date of filing: **29.08.89**

# (54) Halomethyl-1,3,5-triazines containing a monomeric moiety.

(30) Priority: **07.09.88 US 241691**

(43) Date of publication of application: **21.03.90 Bulletin 90/12**

(45) Publication of the grant of the patent: **10.05.95 Bulletin 95/19**

(84) Designated Contracting States: **BE DE FR GB IT NL**

(56) References cited:
**EP-A- 0 109 291**
**DE-A- 2 851 641**
**US-A- 4 758 497**

(73) Proprietor: **MINNESOTA MINING AND MANUFACTURING COMPANY**
**3M Center,**
**P.O. Box 33427**
**St. Paul, Minnesota 55133-3427 (US)**

(72) Inventor: **Bonham, James A. c/o Minnesota Mining and**
**Manufacturing Company**
**2501 Hudson Road**
**St. Paul Minnesota 55144-1000 (US)**
Inventor: **Rossman, Mitchell A. c/o Minnesota Mining and**
**Manufacturing Company**
**2501 Hudson Road**
**St. Paul Minnesota 55144-1000 (US)**
Inventor: **Grant, Richard J. c/o Minnesota Mining and**
**Manufacturing Company**
**2501 Hudson Road**
**St. Paul Minnesota 55144-1000 (US)**

(74) Representative: **Baillie, Iain Cameron et al**
**c/o Ladas & Parry**
**Altheimer Eck 2**
**D-80331 München (DE)**

**EP 0 359 430 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### 1. Field of the Invention

The present invention relates to photosensitive compounds, more particularly, derivatives of halomethyl-1,3,5-triazines.

### 2. Discussion of the Prior Art

Compounds that decompose to generate free radicals (free radical generating agents) upon exposure to light are well known in the graphic arts. Organic halogen compounds, which are capable of generating free radicals such as a chlorine free radical or a bromine free radical upon exposure to light, have been widely used as photoinitiators in photopolymerizable compositions, as photoactivators in free radical photographic compositions, and as photoinitiators for reactions catalyzed by acids formed by light. The spectral sensitivity of these compositions may be broadened by the addition of sensitizers, which, in essence, transfer their absorbed energy to the organic halogen compound. The use of such halogen compounds in photopolymerization processes and free radical photographic processes have been described in Kosar, Light-Sensitive Systems J. Wiley & Sons (New York, 1965), pp. 180-181, 361-370.

Halomethyl-1,3,5-triazines are known to be initiators for a number of photochemical reactions. They are employed to produce free radicals for initiating polymerization or color changes and for initiating secondary reactions upon liberation of acid by the interaction of the free-radicals when hydrogen donors are present.

Examples of the use of halomethyl-1,3,5-triazines in the free radical polymerization of acrylate monomers are described in U.S. Patent No. 3,903,815; U.S. Patent No. 3,617,288; U.S. Patent No. 4,131,752; U.S. Patent No. 4,391,687; U.S. Patent No. 4,475,215; and DE 3,517,440. U.S. Patent. No. 3,779,778 discloses the photoinitiated acid catalyzed decomposition of pyranyl ether derivatives to produce photosolubilizable compositions useful as positive printing plates. Chromophore substituted styryl-1,3,5-triazines and their uses are disclosed in U.S. Patent No. 3,987,037 and U.S. Patent No. 3,954,475. Radiation sensitive compositions containing bi- and polyaromatic substituted triazines are disclosed in U.S. Patent No. 4,189,323.

### SUMMARY OF THE INVENTION

This invention provides radiation-sensitive organo-halogen compounds that have a photo-labile halomethyl-1,3,5-triazine moiety and a polymerizable monomeric moiety within one molecule. The compounds of this invention have at least one halomethyl substituent attached to a carbon atom of the triazine nucleus, preferably a trihalomethyl substituent, and at least one polymerizable monomeric moiety attached to another carbon atom of the triazine nucleus. The polymerizable monomeric moiety is polymerizable by ionic chain polymerization or by free radical polymerization and is selected from acrylamide group, vinyl ether group, allyl ether group, epoxide group, and allyl amine group. In certain situations, it can be polymerizable by active species released from the halomethyl 1-1,3,5-triazine moiety upon exposure of the compound to actinic radiation.

The compounds of this invention are useful in the preparation of polymers having halomethyl-1,3,5-triazine substituents attached thereto. The close proximity of the initiating moiety, i.e., the triazine, to the monomeric moiety increases the efficiency of initiation of polymerization of the compounds of this invention.

The compounds of this invention are good photoinitiators. Photopolymerizable and photocrosslinkable compositions containing them can be used in printing, duplicating, copying, and other imaging systems.

### DETAILED DESCRIPTION OF THE INVENTION

As used herein, the term "monomeric moiety" means a moiety containing at least one group that is capable of undergoing free radical or ionic chain polymerization. Halomethyl substituted, 1,3,5-triazine compounds of this invention can be represented by the general formula I:

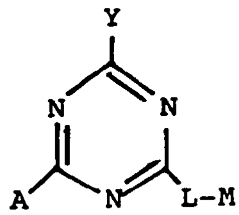

I

wherein

A represents a member selected from the group consisting of mono-, di- and trihalomethyl groups,

Y represents a member selected from the group consisting of A, L-M, $NH_2$, NHR, $NR_2$, OR, and R′, where R independently represents a substituted or unsubstituted alkyl group, preferably having 1 to 6 carbon atoms, or a substituted or unsubstituted aryl group, and R′ represents a substituted or unsubstituted alkyl group, preferably having 1 to 6 carbon atoms, a substituted or unsubstituted aryl group, a substituted or unsubstituted alkenyl group, preferably having 2 to 6 carbon atoms, or a substituted or unsubstituted heterocyclic aromatic group,

M represents a group containing at least one of the above specified monomeric moieties that is capable of undergoing free radical or ionic chain polymerization, and

L represents a group or a covalent bond linking the monomeric moiety to the triazine nucleus.

Halomethyl groups that are suitable for A in the present invention include chloro-, bromo-, and iodomethyl groups, with chloro- and bromomethyl groups being preferred. Trihalomethyl groups are preferred; most preferred are trichloromethyl and tribromomethyl groups.

Y represents any of a variety of substituents that are useful in modifying the physical, e.g., solubility, or chemical properties of the molecule, and preferably represents A, L-M, or R′. When Y represents A, the maximum number of halomethyl groups per triazine nucleus can be made available for free radical generation. When Y represents L-M, the chemical composition for both L-M groups can be the same, or it can be different, depending on the composition of linking group L, monomeric group M, or both. When Y represents R′, and in particular when R′ represents an aryl, aralkenyl, or heterocyclic aromatic group, the spectral sensitivity of the molecule can be varied, based on the photochemical response of R′ to actinic radiation.

When R or R′ represents an aryl group, it is preferred that the group have a maximum or five rings, more preferably three rings, and most preferably one ring. When R or R′ represents a substituted group, the particular identity of the substituents is not critical. However, the substituents should be selected so as not to adversely affect the polymerizability of the compounds of this invention.

Monomeric moieties designated by M can be selected from monomeric groups capable of free-radical or ionic chain polymerization. Preferably, monomeric moieties are selected from the group consisting of acrylates, methacrylates, acrylamides, vinyl ethers, allyl ethers, epoxides, and allyl amines. Most preferred are the acrylates, methacrylates, acrylamides, and vinyl ethers. There is no upper limit on the number of monomeric moieties per triazine nucleus; however, there must be at least one monomeric moiety per triazine nucleus. Preferably, there are one to twelve monomeric moieties per triazine nucleus; more preferably, there are one to six monomeric moieties per triazine nucleus. If more than one monomeric moiety is present per triazine nucleus, they can be from different generic classes or can be different species from the same generic class.

L represents a group that links the monomeric moiety or moieties to the triazine nucleus. The precise identity of L is not critical, but it should be selected so that it does not interfere with or adversely affect the polymerizability or light sensitivity of the compound. L can be formed from a single group or it can be formed from a combination of groups. In addition, L also includes a covalent bond. Groups that are suitable for linking groups include carbamato ($-NHCO_2-$), urea (-NHCONH-), amino (-NH-), amido (-CONH-), aliphatic, e.g., having up to 10 carbon atoms, alkyl, e.g., having up to 10 carbon atoms, haloalkyl, e.g., having up to 10 carbon atoms, alkenyl, e.g., having up to 10 carbon atoms, aryl, e.g., having one ring, styryl, ester ($-CO_2-$), ether (-O-), and combinations thereof. Based on ease of synthesis, the most preferred groups for attachment directly to the triazine nucleus are carbamato, urea, amino, alkenyl, aryl, and ether.

The following list exemplifies typical -L-M group combinations:

$-NHCO_2CH_2CH_2OCOCH=CH_2$

$-NHCO_2CH_2CH_2OCOC(CH_3)=CH_2$

$-NHCO_2CH_2CH=CH_2$

$-NHCONHCH_2CH=CH_2$

$-NHCH_2CH_2OCONHCH_2CH_2OCOCH=CH_2$

$-N(CH_2CH_2OCONHCH_2CH_2OCOCH=CH_2)_2$

$-N(CH_2CH_2OCOCH=CH_2)_2$
$-OCH_2CH=CH_2$
$-N(CH_2CH=CH_2)_2$
$-C_6H_4$-p-$OCOCH=CH_2$
$-C_6H_4$-p-$OCH_2CH_2OCOCH=CH_2$
$-CH=CH-C_6H_4$-p-$OCH_2CH_2OCOCH=CH_2$
$-CH=CH-C_6H_4$-p-$OCH_2CH_2OCOC(CH_3)=CH_2$
$-CH=CH-C_6H_4$-p-$O-CH_2CH_2OCOC(CH_3)_2NHCOC(CH_3)=CH_2$
$-NHCO_2CH_2CH_2CH_2CH_2OCH=CH_2$

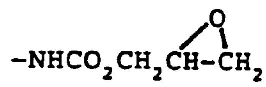

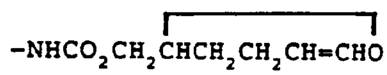

$-CH_2COCH_2CH_2OCH=CH_2$

Representative examples of preferred compounds of this invention have the structures shown in Table I.

## TABLE I

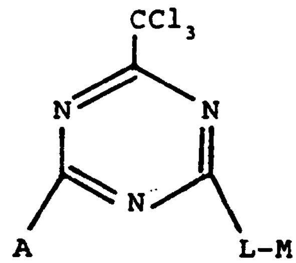

| | A | L-M |
|---|---|---|
| 1 | $-CCl_3$ | $-NH\overset{O}{C}OCH_2CH_2O\overset{O}{C}CH=CH_2$ |
| 2 | $-CCl_3$ | $-NH\overset{O}{C}OCH_2CH_2O\overset{O}{C}C(CH_3)=CH_2$ |
| 3 | $-NH\overset{O}{C}OCH_2CH_2O\overset{O}{C}CH=CH_2$ | $-NH\overset{O}{C}OCH_2CH_2O\overset{O}{C}CH=CH_2$ |
| 4 | $-NH\overset{O}{C}OCH_2CH_2O\overset{O}{C}C(CH_3)=CH_2$ | $-NH\overset{O}{C}OCH_2CH_2O\overset{O}{C}C(CH_3)=CH_2$ |
| 5 | $-CCl_3$ | $-NH\overset{O}{C}OCH_2CH_2CH_2O\overset{O}{C}C(CH_3)=CH_2$ |
| 6 | $-CCl_3$ | $-OCH_2CH=CH_2$ |

| | | |
|---|---|---|
| 7 | $-CCl_3$ | $-NH\overset{O}{\overset{\|}{C}}NHCH_2CH{=}CH_2$ |
| 8 | $-CCl_3$ | $-NH\overset{O}{\overset{\|}{C}}N(CH_2CH{=}CH_2)_2$ |
| 9 | $-CCl_3$ | $-NHCH_2CH{=}CH_2$ |
| 10 | $-CCl_3$ | $-N(CH_2CH{=}CH_2)_2$ |
| 11 | 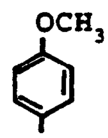 | $-NH\overset{O}{\overset{\|}{C}}OCH_2CH_2O\overset{O}{\overset{\|}{C}}CH{=}CH_2$ |
| 12 | 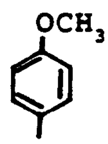 | $-NH\overset{O}{\overset{\|}{C}}OCH_2CH_2O\overset{O}{\overset{\|}{C}}C(CH_3){=}CH_2$ |
| 13 | 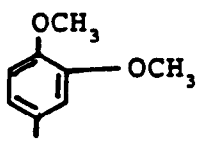 | $-NH\overset{O}{\overset{\|}{C}}OCH_2CH_2O\overset{O}{\overset{\|}{C}}CH{=}CH_2$ |
| 14 | 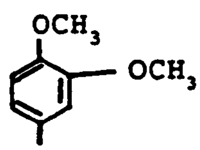 | $-NH\overset{O}{\overset{\|}{C}}OCH_2CH_2O\overset{O}{\overset{\|}{C}}C(CH_3){=}CH_2$ |
| 15 | $-CCl_3$ | 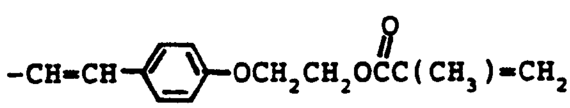 |
| 16 | $-CCl_3$ | 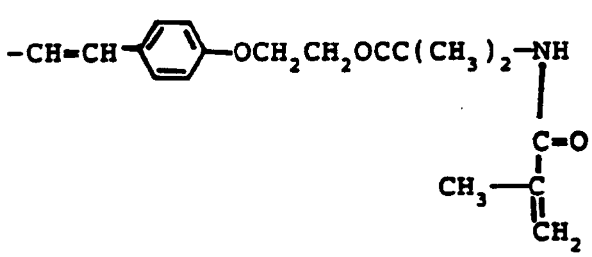 |

| | | |
|---|---|---|
| 17 | $-CCl_3$ | $-NH\overset{O}{\overset{\|}{C}}OCH_2CH_2CH_2CH_2OCH{=}CH_2$ |
| 18 | 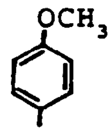 | $-NH\overset{O}{\overset{\|}{C}}OCH_2CH_2CH_2CH_2OCH{=}CH_2$ |
| 19 | $-CCl_3$ | $-NH\overset{O}{\overset{\|}{C}}O-CH_2C(CH_2O\overset{O}{\overset{/\!/}{C}}CH{=}CH_2)_3$ |
| 20 | $-NH\overset{O}{\overset{\|}{C}}OCH_2C(CH_2O\overset{O}{\overset{/\!/}{C}}CH{=}CH_2)_3$ | $-NH\overset{O}{\overset{\|}{C}}OCH_2C(CH_2O\overset{O}{\overset{/\!/}{C}}CH{=}CH_2)_3$ |
| 21 | $-CCl_3$ | 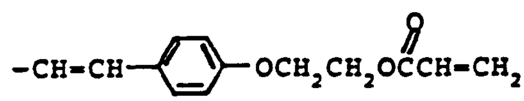 |

From the foregoing table, it should not be inferred that the halomethyl groups suitable for the compounds of this invention are to be limited to $-CCl_3$.

One method of preparing the compounds of this invention is by the addition reaction of isocyanato-substituted halomethyl-1,3,5-triazines with monomeric moiety sources having groups reactive with the isocyanate group. The isocyanato substituted triazines may be prepared from the corresponding amino derivative according to the procedure of U. Von Gizycki, Agnew. Chem. Int. Ed. Eng., 1971, 10, 403. Isocyanato-1,3,5-triazines suitable for this reaction include: 2,4-bis(trichloromethyl)-6-isocyanato-1,3,5-triazine 2-isocyanato-4-methyl-6-trichloromethyl-1,3,5-triazine 2-isocyanato-4-phenyl-6-trichloromethyl-1,3,5-triazine 2-isocyanato-4-methoxy-6-trichloromethyl-1,3,5-triazine 2-isocyanato-4-(p-methoxyphenyl)-6-trichloromethyl-1,3,5-triazine 2-isocyanato-4-(p-methoxystyryl)-6-trichloromethyl-1,3,5-triazine 2-isocyanato-4-(m,p -dimethoxyphenyl)-6-trichloromethyl-1,3,5-triazine

Suitable reagents that contain monomeric moieties and that will combine with the isocyanato group include 2-hydroxyethyl acrylate, 2-hydroxyethyl methacrylate, allyl alcohol, diallyl amines, 2-hydroxyethyl acrylamide, and hydroxybutyl vinyl ether.

The isocyanate addition reaction can be carried out in the presence of solvents, such as, for example, toluene, pyridine, benzene, xylene, dioxane, tetrahydrofuran, etc., and mixtures of solvents. The duration and temperature of the reaction is dependent on the particular compounds and the catalyst employed. Generally, temperatures of about 25 to 150°C for from one to seventy-two hours are sufficient to provide for the reaction. Preferably, the reaction is carried out at room temperature from three to seventy-two hours. The preferred catalyst is di-n-butyltin dilaurate.

Another method for preparing the compounds of this invention involves attaching the monomeric moiety to a group pendant from the triazine nucleus, such as a hydroxyl or amino group. Reagents that are useful in such a method include acryloyl or methacryloyl chloride, 2-isocyanatoethyl methacrylate, and vinyl oxazolinones. The reaction with acryloyl or methacryloyl chloride can be carried out in the presence of solvents, such as, for example, benzene, pyridine, toluene, xylene, etc., and mixtures of solvents. The duration and temperature of the reaction is dependent on the particular compounds and the catalyst for the reaction. Generally, temperatures of about 25 to 150°C for from one to seventy-two hours are sufficient. Preferably, the reaction is carried out at 80-110°C for from three to seventy-two hours. The preferred catalyst is 4-N,N-dimethylaminopyridine. The vinyl oxazolinone reaction is reviewed in Encyclopedia of Polymer Science and Engineering, Vol. 11, edited by J. I. Kroschwitz, Wiley Interscience (New York: 1987), p. 558.

Suitable hydroxyl or amino substituted halomethyl-1,3,5-triazines which can be modified by this method include:
2-amino-4,6-bis(trichloromethyl)-1,3,5-triazine
2-(2-hydroxyethylamino)-4,6-bis(trichloromethyl)-1,3,5-triazine
2-[bis(2-hydroxyethyl)amino]-4,6-bis(trichloromethyl)-1,3,5-triazine
2,4-bis(2-hydroxyethylamino)-6-trichloromethyl-1,3,5-triazine
2,4-bis[bis(2-hydroxyethyl)ethylamino]-6-trichloromethyl-1,3,5-triazine
2-(2-hydroxyethylamino)-4-methoxy-6-trichloromethyl-1,3,5-triazine
2,4-bis(trichloromethyl)-6-(p-hydroxyphenyl)-1,3,5-triazine
2,4-bis(trichloromethyl)-6-(p-hydroxystyryl)-1,3,5-triazine
2,4-bis(trichloromethyl)-6-[p-(2-hydroxyethoxy)phenyl]-1,3,5-triazine
2,4-bis(trichloromethyl)-6-[p-(2-hydroxyethoxy)styryl]-1,3,5-triazine

Another method of preparing the compounds of this invention is the cotrimerization of organic nitriles having a monomeric substituent with haloacetonitriles in accordance with the teachings of Wakabayashi et al, Bulletin of the Chemical Society of Japan, 1969, 42, 2924. Still another method is the condensation reaction of an aldehyde compound having a monomeric functionality in accordance with the teachings of U.S. Patent No. 3,987,037. Another method is the nucleophilic displacement reaction of the halomethyl group of a 1,3,5-triazine using monomers having free hydroxy or amino groups.

The monomer-containing halomethyl-1,3,5-triazines of this invention are particularly useful in photopolymerizable compositions containing ethylenically unsaturated monomeric compounds. A discussion of the free radical polymerization process can be found in Chapter 1 of UV-curing : Science and Technology, Vol. II, edited by S. P. Pappas, Technology Marketing Corporation, Norwalk, Conn. (1985). The initiation step is generally believed to first involve the light induced decomposition of the halocarbon initiator to generate a radical species (e.g. R·) which will add to the double bond of the group containing the monomeric moiety (i.e. M) to form a species (R-M·) necessary for starting the next step of chain propagation. A key factor influencing the efficiency or rate at which the chain propagating species is formed will depend on the ease at which the two species R· and M can combine. The photoinitiators of the invention are expected to perform efficiently in this regard because of the close proximity of each unit, i.e. being within a molecular dimension of one another, in contrast to the situation of requiring the photogenerated radical species R· to diffuse through the composition matrix to a more distant monomer molecule. In actual practice, one must consider other factors in determining to what extent this improvement influences the overall efficiency of a particular photopolymerizable composition. These other factors include initiator concentration, monomer concentration, monomer reactivity, type of binder additives, the degree of inhibition reactions influencing polymer formation by various possible termination steps, and the nature of the final desired effect (e.g. insolubility, hardness, degree of tack, adhesion and others).

The compounds of this invention are also useful as monomeric reagents for the preparation of polymers, copolymers, and composites having halomethyl-1,3,5-triazine substitutents. Polymers containing halomethyl-1,3,5-triazine moieties are useful as photoinitiators and as photocrosslinkable materials. The polymerization reactions may be carried out using the procedures described in G. Odian, Principles of Polymerization, J. Wiley & Sons (New York: 1981) for radical chain or ionic chain polymerization, depending on the specific monomeric substituent. Of particular importance are the compounds of this invention that have one acrylate, methacrylate, or acrylamide substituent. These compounds may be homopolymerized or copolymerized with various other vinyl monomers such as styrene, halogenated olefins, vinyl esters, acrylates, methacrylates, acrylic acid, methacrylic acid, acrylonitrile, and acrylamides. Polymeric materials can be designed to have a wide range of structural and physical properties such as solubility, compatibility, molecular weight, light absorption, etc.

The sensitivity of compositions containing the compounds of this invention to actinic radiation of a particular range of wavelengths can be increased by the incorporation of known ultraviolet and visible light sensitizers including cyanine, carbocyanine, merocyanine, styryl, acridine, polycyclic aromatic hydrocarbons, polyarylamines, and amino-substituted chalcones. Suitable cyanine dyes are described in U.S. Patent No. 3,495,987. Suitable styryl dyes and polyarylamines are described in Kosar, Light Sensitive Systems, J. Wiley and Sons (New York, 1965), pages 361-369. Polycyclic aromatic hydrocarbons useful as sensitizers, an example of which is 2-ethyl-9,10-dimethoxyanthracene, are disclosed in U.S. Patent No. 3,640,718. Amino substituted chalcones useful as sensitizers are described in U.S. Pat. No. 3,617,288. The compounds of this invention can be used in photosensitive compositions in combination with other photoinitiators including benzophenones, benzoin ethers, thioxanthone, benzil, and Michler's ketone. The compounds of this invention can also be substituted for the triazines used in conjunction with dialkylamino aromatic carbonyl compounds disclosed in U.S. Patent No. 4,259,432;
2-(benzoylmethylene)-5-benzothiazolidene thiazole-4-1 compounds disclosed in E application 0109291, May

23, 1984;
3-keto-substituted coumarin compounds disclosed in U.S. Patent No. 4,505,793; U.S. Patent No. 4,239,850; Jpn. Kokai Tokkyo Koho JP 60 60,104 (85 60104); and Ger. Offen. 2,851,641.

Photopolymerizable compositions wherein the compounds of this invention can be used as photoinitiators typically comprise an unsaturated, free radical initiated, chain propagating addition polymerizable compound, a compound of this invention, and optionally one or more fillers, binders, dyes, polymerization inhibitors, color precursors, oxygen scavengers, etc. The compound of this invention should be present in an amount sufficient to initiate polymerization of the polymerizable compound. Examples of suitable ratios of ingredients are as follows: for every 100 parts of polymerizable compound there can be present from 0.005 to 10 parts of photoinitiator, from 0 to 200 parts of filler, from 0 to 200 parts of binder, and from 0 to 10 or more parts of dyes, polymerization inhibitors, color precursors, oxygen scavengers, etc., as may be needed for a particular use of the photopolymerizable compositions. Preferably, there is used per 100 parts of polymerizable compounds 1 to 7.5 parts of the compound of this invention and from 25 to 150 parts of binder.

Unsaturated, free-radical initiated, chain-propagating addition polymerizable compounds suitable for use with the compound of this invention include alkylene or polyalkylene glycol diacrylates, e.g., ethylene glycol diacrylate, diethylene glycol diacrylate, glycerol diacrylate, glycerol triacrylate, ethylene glycol dimethacrylate, 1,3-propanediol dimethacrylate, 1,2,4-butanetriol trimethacrylate, 1,4-cyclohexanediol diacrylate, pentaerythritol tetramethacrylate, pentaerythritol triacrylate, sorbitol hexacrylate; bis[1-(3-acryloxy-2-hydroxy)]-p-propoxyphenyl dimethylmethane, bis[1-(2-acryloxy)]-p-ethoxyphenyldimethylmethane, tris hydroxyethyl-isocyanurate trimethacrylate, the bis-acrylate and the bis-methacrylates of polyethylene glycols of molecular weight 200-500 and the like; unsaturated amides, e.g., methylene bis-acrylamide, methylene bis-methacrylamide, 1,6-hexamethylene bis-acrylamide, diethylene triamine trisacrylamide, beta-methacrylaminoethyl methacrylate; vinyl esters such as divinyl succinate, divinyl adipate, divinyl phthalate. The preferred unsaturated compounds include pentaerythritol tetracrylate, bis[p-(3-acryloxy-2-hydroxypropoxy)phenyl]-dimethylmethane, and bis[p-(2-acryloxyethoxy)phenyl]-dimethylmethane. Mixtures of these esters can also be used as can mixtures of these esters with alkyl esters of acrylic acid and methacrylic acid, including such esters as methyl acrylate, methyl methacrylate, ethyl acrylate, isopropyl methacrylate, n-hexyl acrylate, stearyl acrylate, allyl acrylate, diallyl phthalate, and the like.

To prepare the photosensitive compositions, the components can be admixed in any order and stirred or milled to form a solution or uniform dispersion. Photosensitive elements can be made by coating a photosensitive composition on a suitable base or support and drying the coating. The dry thickness typically ranges from about 0.00005 to about 0.075 inch.

Suitable bases or supports for the photosensitive compositions include metals, e.g., steel and aluminum plates, sheets and foils, and films or plates composed of various film-forming synthetic or high polymers, including addition polymers, e.g., vinylidene chloride, vinyl chloride, vinyl acetate, styrene, isobutylene polymers and copolymers; linear condensation polymers e.g., polyethylene terephthalate, polyhexamethylene adipate, polyhexamethylene adipamide/adipate.

The invention will be more specifically illustrated by the following examples. All values of $\lambda$max were measured in methanol, unless otherwise indicated.

## EXAMPLES 1-6

These examples illustrate the preparation of acrylate-containing the methacrylate-containing halomethyl-substituted-1,3,5-triazine compounds by means of an isocyanate addition reaction.

### Example 1

To a solution comprising 0.008 mol of 2-hydroxyethyl acrylate monomer, 12 drops di-n-butyltin dilaurate, and 100 mg phenothiazine in 30 ml dry toluene (freshly distilled from sodium in the presence of benzophenone) was added a toluene solution containing 0.006 mol 2,4-bis(trichloromethyl)-6-isocyanato-1,3,5-triazine. The reaction mixture was stirred at room temperature under nitrogen atmosphere for 24-72 hrs. The solvent was removed at room temperature by means of a rotary evaporator under reduced pressure. The clear residue was dissolved in a small amount of dichloromethane and loaded upon a silica gel column (100 g packed in (1:1) dichloromethane/hexane) and eluted with dichloromethane. The major product was collected; the appropriate fractions were pooled; and the solvent was removed at room temperature by means of a rotary evaporator to yield the product. The product had a melting point of 115-117°C and $\lambda$max of 235 nm. The structural formula of the product is as follows:

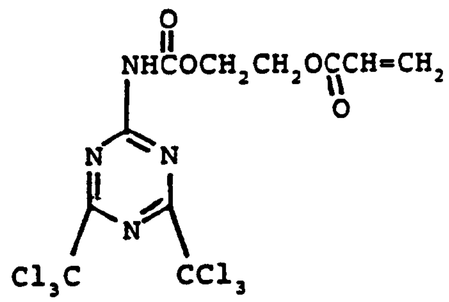

## Example 2

The procedure of Example 1 was repeated with the exception being that 2-hydroxyethyl methacrylate was used instead of 2-hydroxyethyl acrylate. The product had a melting point of 66-69°C and λmax of 235 nm. The structural formula of the product is as follows:

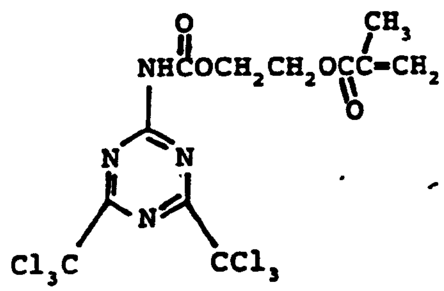

## Example 3

The procedure of Example 1 was repeated with the exception being that 2,4-bis(isocyanato)-6-trichloromethyl-1,3,5-triazine was used instead of 2,4-bis(trichloromethyl)-6-isocyanato-1,3,5-triazine. The product was a gum and had a λmax of 219 nm. The structural formula of the product is as follows:

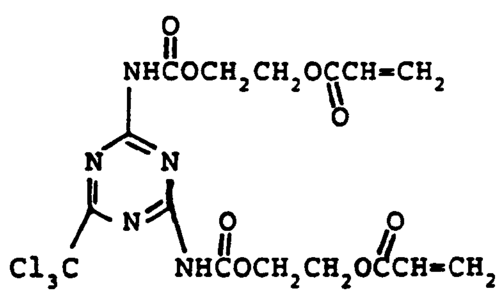

## Example 4

The procedure of Example 2 was repeated with the exception being that 2,4-bis(isocyanato)-6-trichloromethyl-1,3,5-triazine was used instead of 2,4-bis(trichloromethyl)-6-isocyanato-1,3,5-triazine. The product was a gum and had a λmax of 220 nm. The structural formula of the product is as follows:

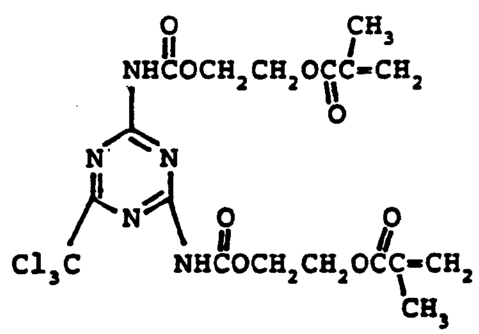

Example 5

The procedure of Example 1 was repeated with the exception being that 2-hydroxypropyl methacrylate was used instead of 2-hydroxyethyl acrylate. The product was a gum and had a λmax of 235 nm (measured in tetrahydrofuran). The structural formula of the product is as follows:

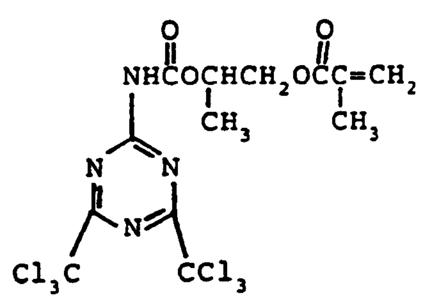

Example 6

The procedure of Example 1 was repeated with the exception being that bis[1-(3-acryloxy-2-hydroxy)]-p-propoxy-phenyl dimethylmethane was used instead of 2-hydroxyethyl acrylate. The product was a gum and had a λmax of 235 nm. The structural formula of the product is as follows:

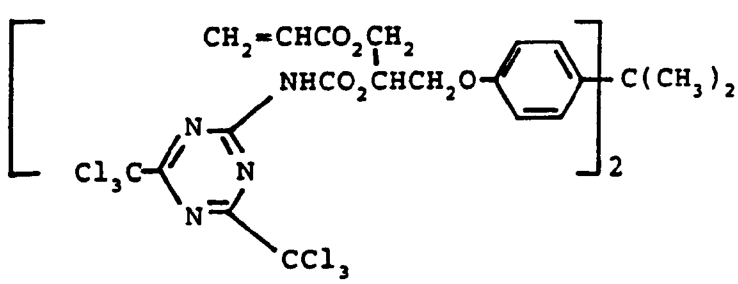

EXAMPLES 7-9

These examples illustrate the preparation of trichloromethyl-1,3,5-triazine compounds containing having an allyl group by means of an isocyanate addition reaction.

Example 7

Using the same procedure as was used in Example 1, 2-isocyanato-4,6-bis(trichloromethyl)-1,3,5-triazine was reacted with allyl alcohol. The product had a melting point of 79-81°C and a λmax of 236 nm (measured in tetrahydrofuran). The structural formula of the product is as follows:

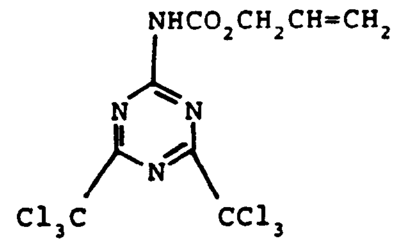

Example 8

Using the same procedure as was used in Example 1, 2-isocyanato-4,6-bis(trichloromethyl)-1,3,5-triazine was reacted with allyl amine. The product had a melting point of 170-172°C and a λmax of 229 nm (measured in tetrahydrofuran). The structural formula of the product is as follows:

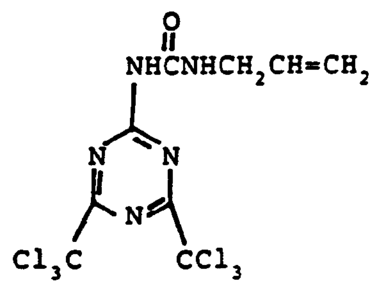

Example 9

Using the same procedure as was used in Example 1, 2-isocyanato-4,6-bis(trichloromethyl)-1,3,5-triazine was reacted with diallyl amine. The product had a melting point of 95-98°C and a λmax of 246 nm (measured in tetrahydrofuran). The structural formula of the product is as follows:

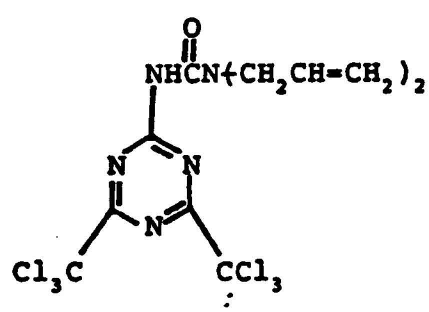

EXAMPLES 10-11

These examples illustrate the preparation of trichloromethyl-1,3,5-triazine compounds containing an allyl group by means of an amine nucleophilic displacement of a trichloromethyl group.

Example 10

To a solution comprising 2.3 mmol 2,4,6-tris(trichloromethyl)-1,3,5-triazine in 25 ml toluene was added 1 equivalent allyl amine. The reaction mixture was stirred at room temperature for 24 hours under a nitrogen atmosphere. The solvent was removed by means of a rotary evaporator under reduced pressure, and the residue was dissolved in a small amount of dichloromethane, loaded upon a column of silica gel (100 g packed in hexane), and eluted with hexane. The appropriate fractions were pooled and the solvent was removed by means of a rotary evaporator to afford product. The product was a gum having a λmax of 246 nm. The structural formula of the product is as follows:

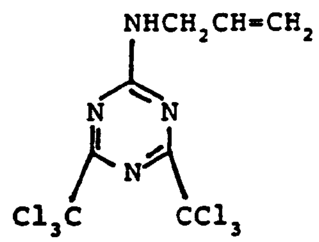

Example 11

The procedure of Example 10 was repeated, with the exception being that diallyl amine was used instead of allyl amine. The product had a melting point of 79-81°C and a λmax of 250 nm (measured in tetrahydrofuran). The structural formula of the product is as follows:

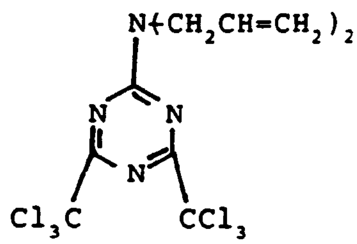

EXAMPLES 12-14

These examples illustrate the preparation of an acrylate, a methacrylate, and a vinyl ether derivative of a trichloromethyl-1,3,5-triazine compound by means of an isocyanate addition reaction.

Example 12

To a solution comprising 0.008 mol of a 2-hydroxyethyl acrylate monomer, 12 drops di-n-butyltin dilaurate, and 100 mg phenothiazine in 30 ml dry benzene (freshly distilled from sodium in the presence of benzophenone) was added a toluene solution containing 0.006 mol 2-isocyanato-4-(p-methoxyphenyl)-6-trichloromethyl-1,3,5-triazine. The reaction mixture was stirred at room temperature under a nitrogen atmosphere for 24-72 hrs. The solvent was removed at room temperature by means of a rotary evaporator under reduced pressure. The clear residue was dissolved in a small amount of dichloromethane, loaded upon a silica gel column (100 g packed in (1:1) dichloromethane/hexane), and eluted with dichloromethane.

The major product was collected; the appropriate fractions were pooled; and the solvent was removed at room temperature by means of a rotary evaporator to yield the product. The product had a melting point of 116-119°C and a λmax of 310 nm (measured in methylene chloride). The structural formula of the product is as follows:

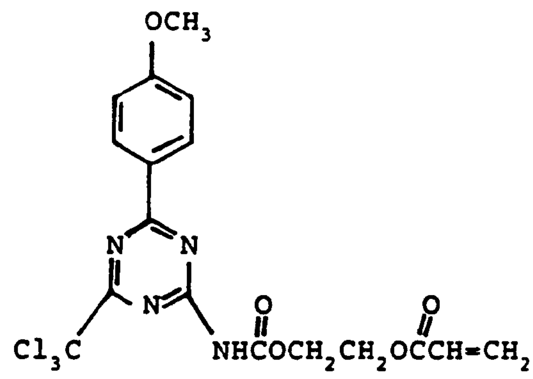

Example 13

The procedure of Example 12 was repeated, with the exception being that 2-hydroxyethyl methacrylate was used instead of 2-hydroxyethyl acrylate. The product had a melting point of 131-133°C and a λmax of 308 nm (measured in methylene chloride). The structural formula of the product is as follows:

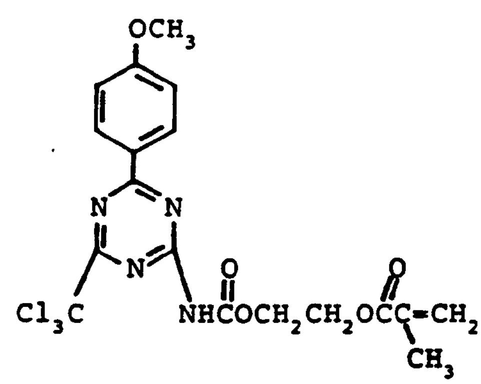

Example 14

The procedure of Example 1 was repeated, with the exception being that 4-hydroxybutylvinylether was used instead of 2-hydroxyethyl acrylate. The product had a melting point of 218-222°C and a λmax of 285 nm (measured in methylene chloride). The structural formula of the product is as follows:

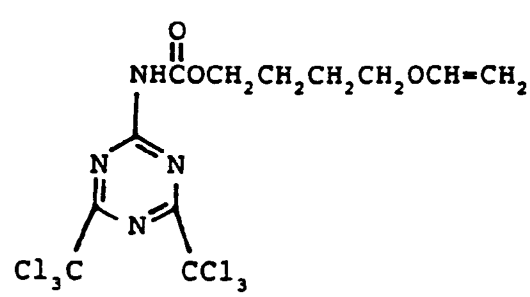

Example 15

This example illustrates the preparation of a methacrylate derivative of a halomethyl substituted-1,3,5-triazine compound by means of the reaction of a hydroxy substituted trichloromethyl-1,3,5-triazine and 2-isocyanatoethyl methacrylate. To a slurry of 1.50 gms of 2,4-bis(trichloromethyl)-6-[p-(2-hydroxyethoxy)styryl]-1,3,5-triazine in 50 ml of toluene/methylene chloride (4:1) was added 0.65 g isocyanatoethyl methacrylate and

three drops di-n-butyltin dilaurate. The reaction was stirred at room temperature overnight and the solvent was removed under reduced pressure by means of a rotary evaporator. The yellow solid was recrystallized with ethanol to yield 1.8 g of the desired product. The product had a melting point of 130-132°C and a λmax of 374 nm. The structural formula of the product is as follows:

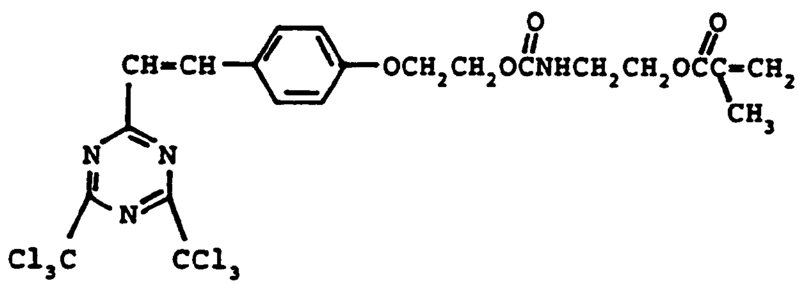

Example 16

This example illustrates the preparation of a methacrylate derivative of a halomethyl substituted-1,3,5-triazine compound by means of the reaction of a hydroxy substituted trichloromethyl-1,3,5-triazine and methacryloyl chloride. To a solution of 1.55 g of 2,4-bis(trichloromethyl)-6-[p-(2-hydroxyethoxy)styryl]-1,3,5-triazine in 50 ml of methylene chloride was added 0.6 g of methacryloyl chloride and 0.6 g of triethyl amine. Upon standing at room temperature for 24 hours, a white solid that had formed, was filtered, and discarded. An additional 50 ml of methylene chloride was added to the filtrate which was then washed twice with solutions of 2N HCl and saturated $NaHCO_3$. After drying with $MgSO_4$, the solvent was removed to give 0.8 g of a yellow oil, which solidified upon tituration with hexane/ethanol and cooling. Recrystallization with absolute ethanol gave mainly the methacrylate ester of the starting triazine with minor impurities. The product had a melting point of 110-118°C and a λmax of 372 nm (measured in ethyl acetate). The structural formula of the product is as follows:

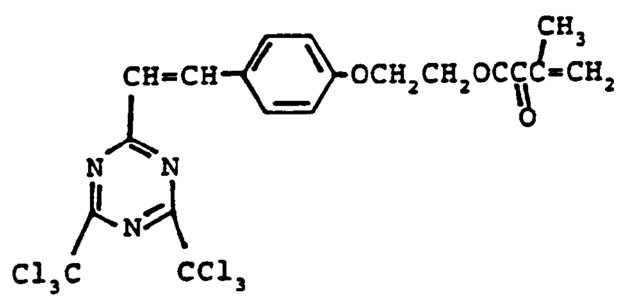

Example 17

This example illustrates the preparation of an acrylamide derivative of a trichloromethyl-1,3,5-triazine compound by means of the reaction of an isopropenyloxazolin-5-one. A solution containing 5.0 g of 2,4-bis(trichloromethyl)-6-[p-(2-hydroxyethoxy)styryl]-1,3,5-triazine, 1.8 g of 2-isopropenyl-4,4-dimethyl-2-oxazolin-5-one, and 0.1 g of 1,8-diazabicyclo-[5.4.0]undec-7-ene in 50 ml of methylene chloride was refluxed for 12 hours and allowed to stand at room temperature for 48 hours. Some insoluble material had formed which was filtered. The methylene chloride was removed under reduced pressure to yield a yellow orange solid containing about 83% of the desired acrylamide product of Example 16 and about 11% of the starting triazine as determined by HPLC. Further purification by recrystallization with ethanol containing a small amount of methylene chloride gave purer product having a melting point of 176-179°C and a λmax of 337 nm (measured in methylene chloride). The structural formula of the product is as follows:

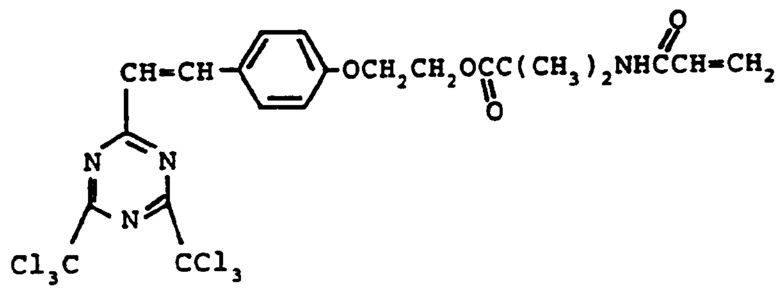

Example 18

This example illustrates the preparation of an dimethacrylate derivative of a trichloromethyl-1,3,5-triazine compound by means of the reaction of a diol and an isocyanatoethyl methacrylate. A solution containing 2.28 g of 2-bis(2-hydroxyethyl)amino-4,6-bis(trichloromethyl)-1,3,5-triazine and 1.69 g of 2-isocyanatoethyl methacrylate in 50 ml of methylene chloride was allowed to stand at room temperature for 4 days. The reaction mixture was then washed with 5% sodium carbonate, water, and dried with $MgSO_4$. After filtering, the solvent was removed under reduced pressure by means of a rotary evaporator to give 3.35 g of the dimethacrylate derivative as a viscous oil having a λmax of 254 nm. The structural formula of the product is as follows:

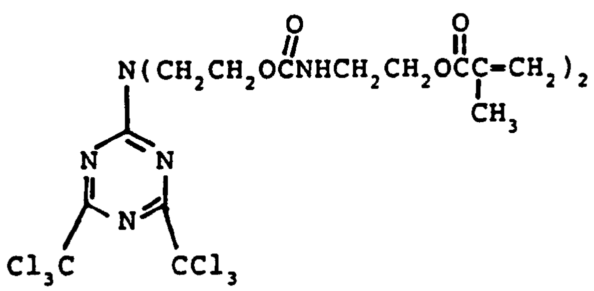

Example 19

This example illustrates the preparation of a methacrylate derivative of a trichloromethyl-1,3,5-triazine compound by means of the cotrimerization of an aromatic nitrile and trichloroacetonitrile. A solution of 5.0 g of 3-cyanophenol, 6.5 g of 2-isocyanatoethyl methacrylate, and 3 drops of di-n-butyltin dilaurate in 50 ml of methylene chloride was allowed to stand at room temperature for 24 hrs. The solution was washed with 20% $Na_2CO_3$, saturated NaCl, dried with $MgSO_4$, and concentrated to give 10.1 g of N-(2-ethylmethacrylate)-(3-cyanophenyl)carbamate having a melting point of 69-71°C.

To a glass pressure vessel was added 2.74 g of the above carbamate, 2.89 g of trichloroacetonitrile, 10 mg of zinc chloride, and 5 ml of methylene chloride. The vessel was connected to a hydrogen chloride cylinder, and then cooled in an acetone-dry ice bath. Hydrogen chloride was added and the system allowed to purge. Upon sealing, a pressure of 20 psi was maintained for one hour; the cooling bath was then removed, and the system was warmed to room temperature. The pressure was raised to 50 psi in the vessel, which was allowed to stand for 5 days. The vessel was opened and 100 ml of methylene chloride added. This solution was extracted with water, dried with $MgSO_4$, and concentrated to give 4.9 g of a viscous oil. When triturated with hexane-methanol a waxy solid formed slowly. Recrystallization with methanol gave the desired product. The product had a melting point of 118-119°C and a λmax of 278 nm. The structural formula of the product is as follows:

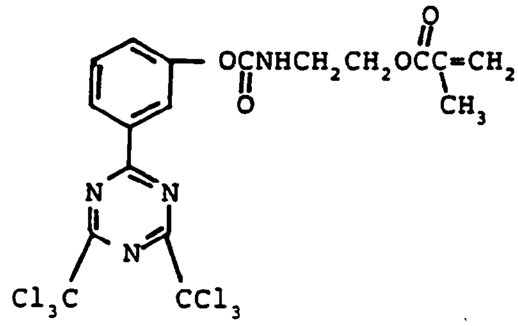

EXAMPLES 20-23

These examples illustrate the preparation of copolymers by using methacrylate derivatives of trichloromethyl-1,3,5-triazine compounds.

Example 20

One part by weight of a triazine monomer (the product of Example 2), nine parts by weight of a co-monomer (isooctyl acrylate), and 0.005 part by weight of azobisisobutyronitrile were dissolved in 15 parts by weight of ethyl acetate and the solution was purged with nitrogen for two to three minutes. The container was capped, placed in a water bath at 50-55°C, and agitated for approximately 20-24 hours. The viscosity of the solution increased and the infrared spectrum of the film forming product showed the disappearance of vinyl monomer. Samples of the polymers were analyzed in a Hewlett Packard 1090 Liquid Chromatograph equipped with a Series L diode array detector and a PLgel 10 micron "mixed" bed column using tetrahydrofuran as the solvent. The elution of the polymer with tetrahydrofuran was followed using the ultraviolet/visible light diode array detector. The spectroscopic data showed that the triazine chromophore was incorporated throughout the polymer. The polymer was isolated from the reaction solution by precipitation with hexane or by evaporation of the solvent. The structural formula of the product is as follows:

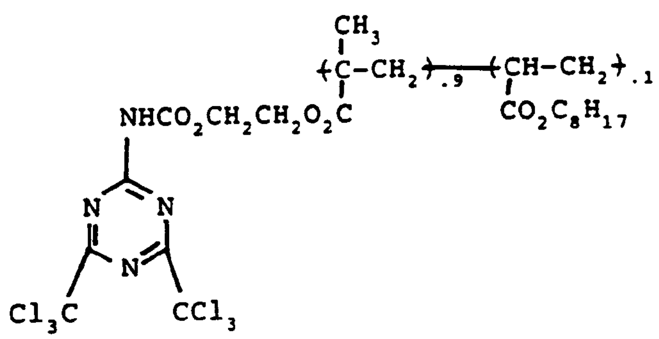

Example 21

The procedure of Example 20 was repeated with the exceptions being that methyl methacrylate was used instead of isooctyl acrylate and that the product of Example 12 was used instead of the product of Example 2. The structural formula of the product is as follows:

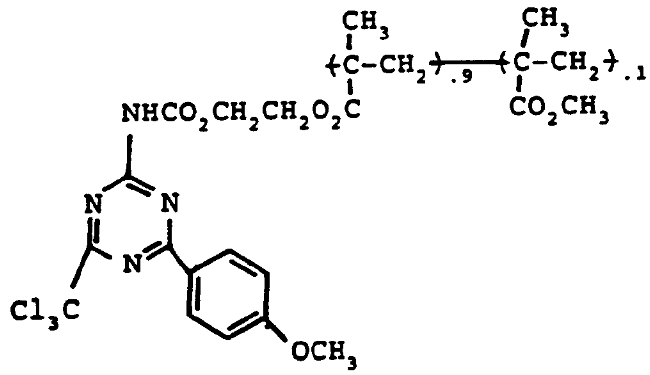

## Example 22

The procedure of Example 20 was repeated with the exceptions being that eight parts by weight of methyl methacrylate was used instead of isooctyl acrylate and that two parts by weight of the product of Example 15 was used instead of the product of Example 2. The structural formula of the product is as follows:

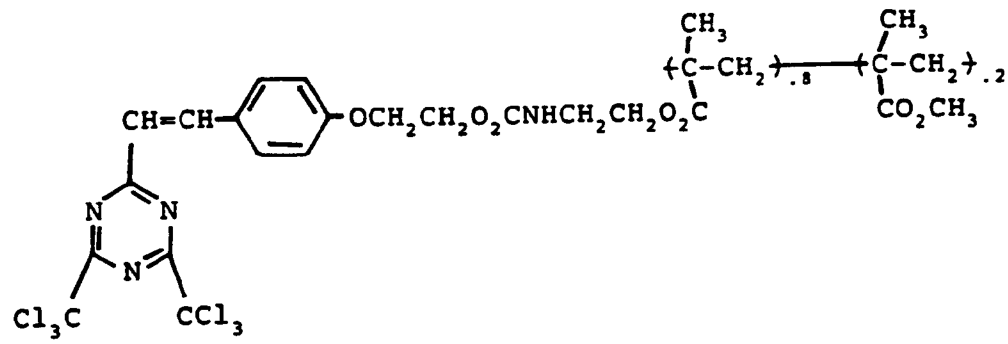

## Example 23

The procedure of Example 20 was repeated with the exception being that the product of Example 19 was used instead of the product of Example 2. The structural formula of the product is as follows:

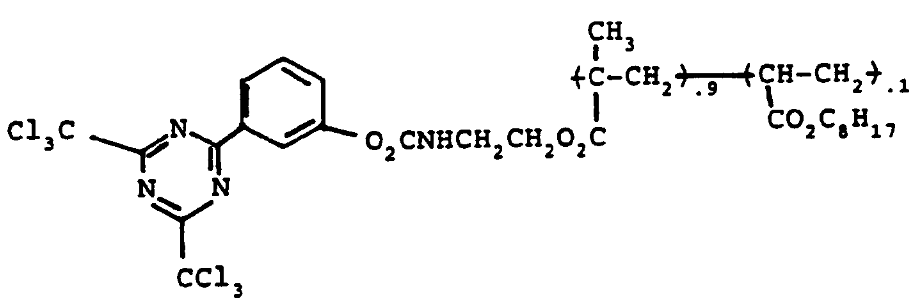

## Example 24

This example illustrates the use of the compounds of this invention in light-sensitive coatings. A solution was prepared from 74.24 g azeotrope of 1-propanol and water (71.8% 1-propanol/28.2% water), 4.32 g pentaerythritol tetraacrylate ("Sartomer" monomer SR-295, Arco Chemical Company), 5.64 g oligomer (prepared according to U.S. Patent No. 4,228,232; 60.9% in methyl ethyl ketone), 0.30 g triethylamine, and 14.88 g a 1:1 mixture of polyvinyl acetate-methylal resin ("Formvar" 12/85T, Union Carbide Corp.) and red pigment (Pigment Red 48, C.I. 15865) (9.4% by weight solution of the azeotrope). To 2.5 g of this solution was added 2.5 mg dimethylaminobenzylace tone (DMBA), 10 mg initiator, and the resulting solution shaken in the dark for 15 minutes. The solution was then filtered through glass wool and coated onto a grained, anodized aluminum plate with a #12 Mayer bar. The plate was dried at 66°C for 2 min and then cooled to room temperature. To this coating was applied a topcoat formulation (prepared from 5.00 g carboxymethyl cellulose ether (CMC-7L), 0.26 g surfactant ("Triton" X-100; 10% in water), and 95.00 g water) wth a #14 Mayer bar and carefully dried with a heat gun. The plates were exposed for 5 sec in air on top of a draw-down glass in a 3M Seventy exposure unit equipped with a 2 kw photopolymer bulb through a √2, 21 step Stouffer step tablet. The plates were soaked in a developer solution prepared from 784.4 g deionized water, 16.7 g sodium metasilicate pentahydrate, 33.4 g 1-propanol, and 0.5 g surfactant ("Dowfax-2A1", Dow Chemical Company) (45% solution in water) for 15 sec and rubbed 10 times with a 4 in. x 4 in. cotton pad. The relative sensitivities for the triazine compounds of Examples 1-5, 7-11 are shown in Table 2.

Table 2

| Initiator | Solid step no. |
|---|---|
| Example 1 | 12 |
| Example 2 | 13 |
| Example 3 | 6 |
| Example 4 | 6 |
| Example 5 | 13 |
| Example 7 | 13 |
| Example 8 | 11 |
| Example 9 | 12 |
| Example 10 | 10 |
| Example 11 | 11 |

Various modifications and alterations of this invention will become apparent to those skilled in the art without departing from the scope and spirit of this invention, and it should be understood that this invention is not to be unduly limited to the illustrative embodiments set forth herein.

## Claims

1. A 1,3,5-triazine compound having at least one halomethyl substituent attached to a carbon atom on the triazine nucleus and at least one polymerisable monomeric moiety attached to a different carbon atom of the triazine nucleus, said polymerisable monomeric moiety being capable of free radical or ionic chain polymerisation and being selected from the group consisting of acrylamide group, vinyl ether group, allyl ether group, epoxide group, and allyl amine group.

2. A compound according to claim 1 wherein said at least one halomethyl group is a trihalomethyl group.

3. A compound according to claim 2 wherein said trihalimethyl group is a member selected from the group consisting of trichloromethyl group, tribromomethyl group, and triiodomethyl group.

4. A compound according to any of claims 1 to 3 having the formula:

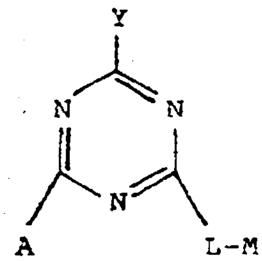

wherein

A represents a member selected from the group consisting of mono-, di- and trihalomethyl groups,

Y represents a member selected from the group consisting of A, L-M, $NH_2$, NHR, $NR_2$, OR, and R′, where R independently represents a substituted or unsubstituted alkyl group, or a substituted or unsubstituted aryl group, R′ represents a substituted or unsubstituted alkyl group, a substituted or unsubstituted aryl group, a substituted or unsubstituted alkenyl group, or a substituted or unsubstituted heterocyclic aromatic group,

M represents said at least one monomeric moiety capable of undergoing free radical or ionic chain polymerisation, and

L represents a group or a covalent bond linking the monomeric moiety to the triazine nucleus.

5. A compound according to claim 4 wherein Y represents A.

6. A compound according to claim 4 wherein Y represents L-M.

7. A compound according to any of claims 4 to 6 wherein R′ represents a substituted or unsubstituted aryl group.

8. A compound according to any of claims 4 to 6 wherein R′ represents a substituted or unsubstituted heterocyclic aromatic group.

9. A compound according to any of claims 4 to 6 wherein R′ represents a substituted or unsubstituted alkenyl group.

10. A radiation-sensitive composition comprising: (1) an ethylenically unsaturated polymerisable compound, and (2) a photoinitiator compound having the formula as defined in claim 4.

## Patentansprüche

1. 1,3,5-Triazin-Verbindung mit mindestens einem Halogenmethyl-Substituenten an einem Kohlenstoffatom im Triazin-Ring und mindestens einem polymerisierbaren monomeren Teil an einem anderen Kohlenstoffatom des Trizin-Ringes, welcher polymerisierbare monomere Teil eine Radikal- oder Ionenkettenpolymerisation eingehen kann und ausgewählt wird aus der Gruppe, bestehend aus einer Acrylgruppe, Vinylether-Gruppe, Allylether-Gruppe, Epoxid-Gruppe und Allylamin-Gruppe.

2. Verbindung nach Anspruch 1, bei welcher die mindestens eine Halogenmethyl-Gruppe eine Trihalogenmethyl-Gruppe ist.

3. Verbindung nach Anspruch 2, bei welcher die Trihalogenmethyl-Gruppe ein Vertreter ist, ausgewählt aus der Gruppe, bestehend aus Trichlormethyl, Tribrommethyl- und Triiodmethyl-Gruppe.

4. Verbindung nach Anspruch 1 bis 3 mit der Formel

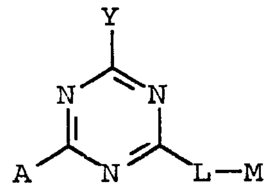

worin sind:

A ein Vertreter, ausgewählt aus der Gruppe, bestehend aus Mono-, Di- und Trihalogenmethyl-Gruppen;

Y ein Vertreter, ausgeweälht aus der Gruppe bestehend aus A, L-M, $NH_2$, NHR, $NR_2$, OR und R′, worin R unabhängig eine substituierte oder nichtsubstituierte Alkyl-Gruppe oder eine substituierte oder nichtsubstituierte Aryl-Gruppe darstellt, R′ eine substituierte oder nichtsubstituierte Alkyl-Gruppe oder eine substituierte oder nichtsubstituierte Aryl-Gruppe, eine substituierte oder nichtsubstituierte Alkenyl-Gruppe oder eine substituierte oder nichtsubstituierte heterocyclische aromatische Gruppe,

M den mindestens einen monomeren Teil, der eine Radikal- oder Ionenkettenpolymerisation eingehen kann, und

L eine Gruppe oder kovalente Bindung, welche den monomeren Teil mit dem Triazin-Ring verknüpft.

**5.** Verbindung nach Anspruch 4, bei welcher Y A darstellt.

**6.** Verbindung nach Anspruch 4, bei welcher Y L-M darstellt.

**7.** Verbindung nach Anspruch 4 bis 6, bei welcher R′ eine substituierte oder nichtsubstituierte Aryl-Gruppe darstellt.

**8.** Verbindung nach Anspruch 4 bis 6, bei welcher R′ eine substituierte oder nichtsubstituierte heterocyclische aromatische Gruppe darstellt.

**9.** Verbindung nach Anspruch 4 bis 6, bei welcher R′ eine substituierte oder nichtsubstituierte Alkenyl-Gruppe darstellt.

**10.** Strahlungsempfindliche Zusammensetzung, umfassend: (1) eine ethylenisch ungesättigte, polymerisierbare Verbindung und (2) eine Photoinitiator-Verbindung mit der in Anspruch 4 festgelegten Formel.

## Revendications

**1.** Composé de 1,3,5-triazine comportant au moins un substituant halométhyle fixé à un atome de carbone sur le noyau triazine et au moins un fragment monomère polymérisable fixé à un atome de carbone différent du noyau triazine, le fragment monomère polymérisable susdit pouvant subir une polymérisation radicalaire ou du type chaîne ionique et étant choisi dans le groupe comprenant un groupe acrylamide, un groupe éther vinylique, un groupe éther allylique, un groupe époxyde et un groupe amine allylique.

**2.** Composé suivant la revendication 1, dans lequel au moins le groupe halométhyle précité est un groupe trihalométhyle.

**3.** Composé suivant la revendication 2, dans lequel le groupe trihalométhyle est un membre choisi dans le groupe comprenant le groupe trichlorométhyle, le groupe tribromométhyle et le groupe triiodométhyle.

**4.** Composé suivant l'une quelconque des revendications 1 à 3, ayant la formule :

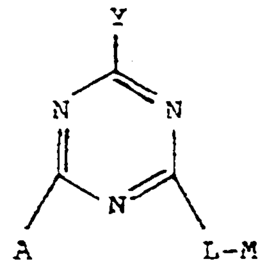

dans laquelle :

A représente un membre du groupe comprenant les groupes mono-, di- et trihalométhyle,

Y représente un membre du groupe comprenant A, L-M, $NH_2$, NHR, $NR_2$, OR et R′, où R représente indépendamment un groupe alkyle substitué ou non substitué ou un groupe aryle substitué ou non substitué, R′ représente un groupe alkyle substitué ou non substitué, un groupe aryle substitué ou non substitué, un groupe alcényle substitué ou non substitué ou un groupe aromatique hétérocyclique substitué ou non substitué,

M représente au moins le fragment monomère précité pouvant subir une polymérisation radicalaire ou du type chaîne ionique, et

L représente un groupe ou une liaison covalente reliant le fragment monomère au noyau triazine.

**5.** Composé suivant la revendication 4, dans lequel Y représente A.

**6.** Composé suivant la revendication 4, dans lequel Y représente L-M.

**7.** Composé suivant l'une quelconque des revendications 4 à 6, dans lequel R′ représente un groupe aryle substitué ou non substitué.

**8.** Composé suivant l'une quelconque des revendications 4 à 6, dans lequel R′ représente un groupe aromatique hétérocyclique substitué ou non substitué.

**9.** Composé suivant l'une quelconque des revendications 4 à 6, dans lequel R′ représente un groupe alcényle substitué ou non substitué.

**10.** Composition radiosensible comprenant :

(1) un composé polymérisable éthyléniquement insaturé et

(2) un composé photoinitiateur répondant à la formule telle que définie à la revendication 4.